(19)

Europäisches
Patentamt
European
Patent Office
Office européen
des brevets

(11) **EP 2 169 621 A1**

(12) **EUROPEAN PATENT APPLICATION**
published in accordance with Art. 153(4) EPC

(43) Date of publication:
**31.03.2010 Bulletin 2010/13**

(51) Int Cl.:
***G06T 7/00*** *(2006.01)*

(21) Application number: **08778226.4**

(22) Date of filing: **10.07.2008**

(86) International application number:
**PCT/JP2008/062875**

(87) International publication number:
**WO 2009/008550 (15.01.2009 Gazette 2009/03)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **11.07.2007 JP 2007182631**

(71) Applicant: **Sony Corporation
Tokyo 108-0075 (JP)**

(72) Inventor: **ABE, Hiroshi
Tokyo 180-0075 (JP)**

(74) Representative: **Merryweather, Colin Henry et al
J.A. Kemp & Co.
14 South Square
Gray's Inn
London WC1R 5JJ (GB)**

(54) **REGISTERING APPARATUS, COLLAITING APPARATUS, DATA STRUCTURE, AND STORAGE MEDIUM**

(57)    A registration apparatus, a checking apparatus, a data structure, and a storage medium that are capable of achieving an improved authentication accuracy are provided. The registration apparatus includes an image acquisition unit configured to acquire a venous image for a vein of a living body, an extraction unit configured to extract a parameter resistant to affine transformation from part of the venous image, and a registration unit configured to register the parameter extracted by the extraction unit in storage means. The part of the venous image is set as a target for extracting the parameter resistant to affine transformation.

FIG. 10

(A)  (B)  (C)

$I_1 = 2.44 \times 10^{-3}$  $I_1 = 2.59 \times 10^{-3}$  $I_1 = 1.92 \times 10^{-3}$
$I_2 = 6.36 \times 10^{-7}$  $I_2 = 6.19 \times 10^{-7}$  $I_2 = 3.40 \times 10^{-7}$

EP 2 169 621 A1

**EP 2 169 621 A1**

**Description**

Technical Field

The present invention relates to a registration apparatus, a checking apparatus, a data structure, and a storage medium and is suited for, for example, application to biometrics authentication.

Background Art

Authentication technologies employing a living body as an authentication target are coming into wide use. And, incorporating biometric authentication into a potable communication device, such as a cellular phone, enables authentication processing to be easily performed on a communication party anywhere through the portable communication device, so it is important to incorporate a biometric authentication apparatus into a portable communication device.

Traditionally, one example of biometric authentication apparatuses is a vein authentication apparatus employing a vein of a finger as a target. The vein authentication apparatus generates pattern information indicating a vein characteristic in a venous image obtained as a result of image pickup inside a finger, and registers it into storage means or checks it against pattern information registered in the storage medium.

Meanwhile, when pattern information is registered and when it is checked against registered pattern information, if a misalignment of a venous section with respect to an image pickup unit occurs, an event in which a registrant is falsely determined as an unregistered person or an event in which an unregistered person is falsely determined as a registrant occurs, so a low authentication accuracy problem exists. One solution to this problem is the one in which a waveform state of luminance histogram is utilized as pattern information to prevent incorrect determination resulting from a misalignment occurring in an image pickup (refer to Patent Document 1, for example).

Patent Document 1: Japanese Unexamined Patent Application Publication No. 2005-312748

However, for this document, for example, at authentication, if a misalignment occurs in a state where even part of a venous section at registration is outside an image pickup range, an obtained waveform state of luminance histogram is significantly different from one at registration, so a low authentication accuracy problem exists.

Disclosure of Invention

The present invention is made in consideration of the above respects and is directed to providing a registration apparatus, a checking apparatus, a data structure, and a storage medium that are capable of achieving an improved authentication accuracy.

To solve the above problems, the present invention is a registration apparatus that includes an image acquisition unit configured to acquire a venous image for a vein of a living body, an extraction unit configured to extract a parameter resistant to affine transformation from part of the venous image, and a registration unit configured to register the parameter extracted by the extraction unit in storage means.

And, the present invention is an authentication apparatus an extraction unit configured to extract a parameter resistant to affine transformation from part of a venous image for a vein of a living body, the venous image being input as an authentication target, a reading unit configured to read registration information registered in storage means, and a determination unit configured to determine whether a person who input the parameter is a registrant in accordance with a degree of checking between the parameter and the registration information.

In addition, the present invention is a data structure for use in processing of determination of a registrant or not performed by a computer. The data structure includes a parameter extracted from part of a venous image for a vein of the registrant, the parameter being resistant to affine transformation. In the processing, the parameter is compared with a corresponding section in a venous image input as an authentication target.

In addition, the present invention is a storage medium that stores data for use in processing of determination of a registrant or not performed by a computer. The data includes a parameter extracted from part of a venous image for a vein of the registrant, the parameter being resistant to affine transformation. In the processing, the parameter is compared with a corresponding section in a venous image input as an authentication target.

As described above, with the present invention, because a target for extracting a parameter resistant to affine transformation is part of a venous image, the permissible amount of movement of a vein within a venous image can be maintained without an increase in an image pickup range. In this manner, a registration apparatus, a checking apparatus, a data structure, and a storage medium that are capable of achieving an improved authentication accuracy can be accomplished.

Brief Description of Drawings

Fig. 1 is a block diagram that illustrates a configuration of an authentication apparatus according to the present embodiment.
Fig. 2 is a block diagram that illustrates a functional configuration of a control unit in vascular registration mode.
Fig. 3 is a block diagram that illustrates a functional configuration of the control unit in authentication mode.
Fig. 4 is a block diagram that illustrates an image processing unit.
Fig. 5 is schematic diagrams that illustrate images before and after sharpening processing.
Fig. 6 is schematic diagrams that illustrate sample images.
Fig. 7 is schematic diagrams that illustrate states of rotational movement and translational movement.
Fig. 8 is schematic diagrams that illustrate relationships between moment invariants and degrees.
Fig. 9 is a schematic diagram that illustrates a range for extracting moment invariants.
Fig. 10 is schematic diagrams that illustrate relationships between movement and moment invariants.
Fig. 11 is schematic diagrams for use in describing a size of an image pickup range whose center lies in a diverging point.
Fig. 12 is a schematic diagram for use in describing a diverging point to be a reference for extracting moment invariants.
Fig. 13 is a schematic diagram that illustrates a data structure.
Fig. 14 is a block diagram that illustrates a configuration of a determination unit.
Fig. 15 is schematic diagrams that illustrate image examples of a range for extracting moment invariants and a diverging point being a reference for that range.
Fig. 16 is a schematic diagram that illustrates comparison of moment invariants.
Fig. 17 is a schematic diagram for use in describing a geometric correlation.
Fig. 18 is a flowchart that illustrates a procedure of authentication processing.

Best Modes for Carrying Out the Invention

An embodiment in which the present invention is applied is described below about the drawings.

(1) General Configuration of Authentication Apparatus

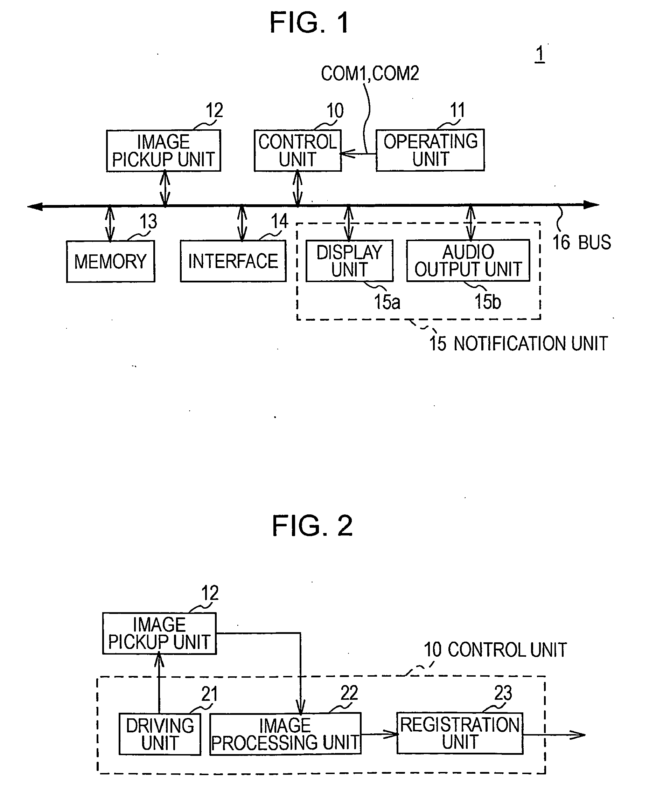

Fig. 1, a general configuration of an authentication apparatus 1 according to the present embodiment is described. The authentication apparatus 1 is configured such that an operating unit 11, an image pickup unit 12, a memory 13, an interface 14, and a notification unit 15 are connected to a control unit 10 through a bus 16.
The control unit 10 is configured as a computer containing a central processing unit (CPU) controlling the whole of the authentication apparatus 1, a read-only memory (ROM) in which various programs and setting information are stored, and a random-access memory (RAM) serving as a work memory of the CPU.
In response to a user operation, a command COM1 to execute a mode for registering a vein of a user being a registration target (hereinafter, the mode is referred to as a vein registration mode, and the registration target user is referred to as a registrant) or a command COM2 to execute a mode for determining whether a person is a registrant (hereinafter referred to as an authentication mode) is input from the operating unit 11 into the control unit 10.
The control unit 10 determines a mode to be executed on the basis of the command COM1 or COM2, appropriately controls the image pickup unit 12, the memory 13, the interface 14, and the notification unit 15 on the basis of a program corresponding to a result of the determination, and executes the vein registration mode or the authentication mode.
The image pickup unit 12 emits light that has a wavelength contained in a wavelength range (700 [nm] to 900 [nm]) having a characteristic of being specifically absorbed in both deoxygenated hemoglobin and oxygenated hemoglobin (hereinafter, this light is referred to as near-infrared light) on a surface being a target on which a finger is to be placed (hereinafter referred to as a finger placement surface).
And, the image pickup unit 12 acquires an image in which a vein within a living-body portion placed on a finger placement surface is projected (hereinafter referred to as a venous image) as data on the image (hereinafter referred to as venous image data) and outputs the venous image data to the control unit 10.
The memory 13 can be a flash memory, for example, and stores or reads data specified by the control unit 10.
The interface 14 exchanges various kinds of data with an external device connected through a predetermined transmission line.
The notification unit 15 includes a display unit 15a and an audio output unit 15b. The display unit 15a displays text and graphics based on display data supplied from the control unit 10. On the other hand, the audio output unit 15b outputs, from a speaker, audio based on audio data supplied from the control unit 10.

(1-1) Vein Registration Mode

Next, the vein registration mode is described. When determining the vein registration mode as the mode to be executed, the control unit 10 notifies that a finger should be placed on the finger placement surface through the notification unit 15, and after that, as illustrated in Fig. 2, the control unit 10 functions as a driving unit 21, an image processing unit 22, and a registration unit 23.

In this case, the driving unit 21 acquires venous image data by driving the image pickup unit 12. That is, the driving unit 21 emits near-infrared light on the finger placement surface by driving a light source of the image pickup unit 12. And, the driving unit 21 adjusts a lens position of an optical lens of the image pickup unit 12 such that it is focused on a subject. In addition, the driving unit 21 adjusts an f-number of a stop of the image pickup unit 12 on the basis of a predetermined exposure value (EV) and adjusts a shutter speed (exposure time) with respect to an image pickup element.

The image processing unit 22 extracts, as a feature of a venous image, a parameter resistant to affine transformation from venous image data supplied from the image pickup unit 12 as a result of image pickup in the image pickup unit 12. The parameter resistant to affine transformation is a parameter that is invariant even if the position changes when a luminance state in an image is invariant. Hereinafter, this parameter is referred to as a feature as appropriate.

The registration unit 23 generates data for indentifying a registrant (hereinafter referred to as registration data) on the basis of a feature extracted by the image processing unit 22 and registers it by storing it into the memory 13.

In such a way, the control unit 10 can execute the vein registration mode.

(1-2) Authentication Mode

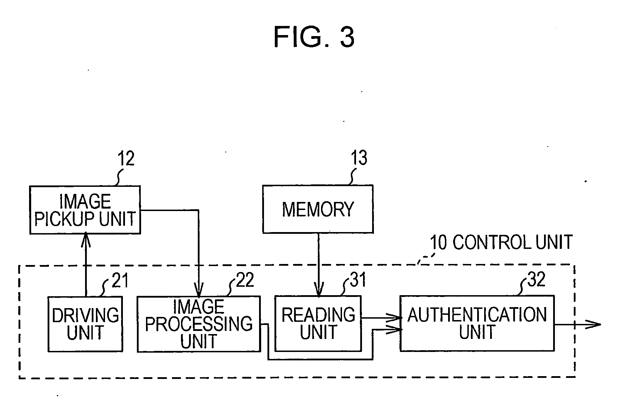

Next, the authentication mode is described. When determining the authentication mode as the mode to be executed, the control unit 10 notifies that a finger should be placed on the finger placement surface through the notification unit 15, and after that, as illustrated in Fig. 3, in which like parts are identified by the same reference numerals as in Fig. 2, the control unit 10 functions as the driving unit 21, the image processing unit 22, a reading unit 31, and an authentication unit 32.

In this case, the driving unit 21 drives the image pickup unit 12, and the image processing unit 22 extracts a feature on the basis of venous image data supplied from the image pickup unit 12.

The reading unit 31 acquires registration data by reading it from the memory 13 and supplies it to the authentication unit 32.

The authentication unit 32 checks the feature of the registration data supplied from the reading unit 31 against the feature extracted by the image processing unit 22 and determines whether a provider of the venous image data can be authenticated as a registrant in accordance with the degree of the checking.

When determining that the provider cannot be authenticated as a registrant, the authentication unit 32 visually and aurally notifies that the provider cannot be authenticated through the display unit 15a and the audio output unit 15b.

On the other hand, when determining that the provider can be authenticated as a registrant, the authentication unit 32 sends data for starting execution of predetermined processing to a device connected to the interface 14. The device performs, as the predetermined processing, processing to be executed for successful authentication, for example, closing a door in a fixed period of time or clearing an operational mode of a restriction target.

In such a way, the control unit 10 can execute the authentication mode.

(2) Details of Processing in Image Processing Unit

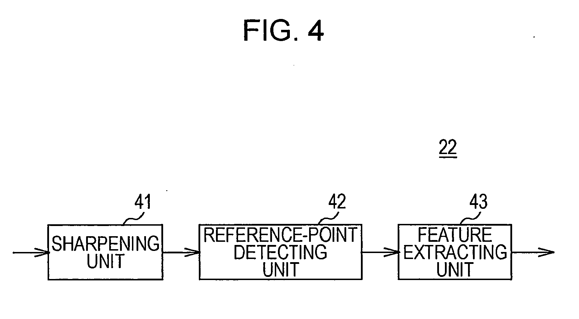

Next, the image processing unit 22 is specifically described. The image processing unit 22 has a configuration including a sharpening unit 41, a reference-point detecting unit 42, and a feature extracting unit 43, as illustrated in Fig. 4.

(2-1) Sharpening Processing

The sharpening unit 41 performs sharpening processing called LoG filtering on venous image data obtained from the image pickup unit 12 (Fig. 2) and thereby makes a vein contained in the venous image stand out in sharp relief.

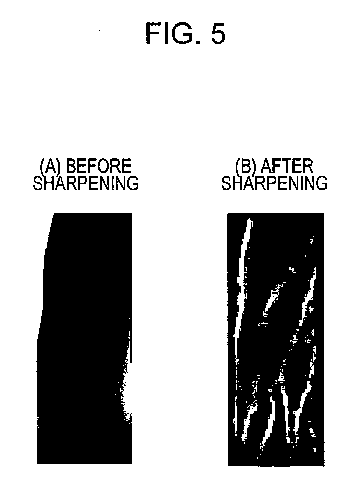

Here, images before and after sharpening processing are illustrated in Fig. 5. As is clear from this Fig. 5, sharpening processing makes the difference (border section) between the vein and the background prominent, and as a result, the vein contained in the venous image is made to stand out from the background.

(2-2) Reference-Point Detection Processing

The reference-point detecting unit 42 detects a diverging point of a vein contained in a venous image on the basis of venous image data supplied from the sharpening unit 41.

In the present embodiment, the reference-point detecting unit 42 binarizes a venous image and extracts the center of a vein width or the peak of luminance in the binarized image, and thereby generates a pattern in which the vein line width corresponds to one pixel, as pre-stage processing for detecting a diverging point. Accordingly, the reference-point detecting unit 42 can obtain more uniformly detect the position of an intersection of veins, as compared with the case where the pre-stage processing is omitted.

(2-3) Feature Extraction Processing

The feature extracting unit 43 acquires venous image data after sharpening processing from the sharpening unit 41 and calculates a feature of the venous image. The feature extracting unit 43 uses a moment invariant as the feature.

A moment invariant is briefly described. An image moment of the order (p+q) of an image f(x, y) at the coordinates (x, y) represents a variance value of a pixel whose center lies in the origin of an image, as defined by the following expression:

$$m_{pq} = \sum_{y=1}^{h} \sum_{x=1}^{w} x^p y^q f(x,y) \qquad \cdots\cdots \ (1)$$

Accordingly, as a large pixel value is farther dispersed from the origin, the image moment has a larger value.

In this Expression (1), "p" represents a weight with respect to the x-axis direction, and "q" represents a weight with respect to the y-axis direction. Accordingly, as the value of "p" increases, the weight with respect to the variance toward the x-axis direction increases, whereas as the value of "q" increases, the weight with respect to the variance toward in the y-axis direction increases. Incidentally, in Expression (1), "w" represents the number of pixels in the x-axis direction, and "h" represents the number of pixels in the y-axis direction.

For this image moment, $m_{10}/m_{00}$ and $m_{01}/m_{00}$ represent the barycentric coordinates $G(x_G, y_G)$. On the basis of this barycentric coordinates $G(x_G, y_G)$, the moment around the barycenter, i.e., barycentric moment can be defined by the following expression:

$$M_{pq} = \sum_{y=1}^{h} \sum_{x=1}^{w} (x - x_G)^p (y - y_G)^q f(x,y) \qquad \cdots\cdots \ (2)$$

This is a moment in which the distance from the barycenter is considered, not the distance from the origin. That is, this value represents variance in which the barycenter with respect to each of the x-axis direction and the y-axis direction in a pixel value within an image is considered.

In addition, this barycentric moment is normalized by the following expressions:

$$\eta_{pq} = \frac{{}_G m_{pq}}{\left({}_G m_{00}\right)^\gamma}$$

$$\gamma = \frac{p+q}{2} + 1, \qquad \text{for} , \ (p+q) \geq 2 \qquad \cdots\cdots \ (3)$$

and thus the normalized barycentric moment $\eta$ is calculated. Due to this normalization, the spread of variance does not affect the value of moment, so it is invariant with respect to translation movement, rotation movement of an object within an image, or an image size.

Moment invariants are ones in which these normalized barycentric moments are combined and can be classified into seven types of the first ($I_1$) to seventh ($I_7$) orders, as defined by the following expressions:

$$I_1 = \eta_{20} + \eta_{02}$$

$$I_2 = (\eta_{20} - \eta_{02})^2 + 4\eta_{11}^2$$

$$I_3 = (\eta_{30} - 3\eta_{12})^2 + (3\eta_{21} - \eta_{03})^2$$

$$I_4 = (\eta_{30} + \eta_{12})^2 + (\eta_{21} + \eta_{03})^2$$

$$I_5 = (\eta_{30} - 3\eta_{12})(\eta_{30} + \eta_{12})\left\{(\eta_{30} + \eta_{12})^2 - 3(\eta_{21} + \eta_{03})^2\right\} +$$

$$(3\eta_{21} - \eta_{03})(\eta_{21} + \eta_{03})\left\{3(\eta_{30} + \eta_{12})^2 - (\eta_{21} + \eta_{03})^2\right\}$$

$$I_6 = (\eta_{20} - \eta_{02})\left\{(\eta_{30} + \eta_{12})^2 - (\eta_{21} + \eta_{03})^2 + 4\eta_{11}(\eta_{30} + \eta_{12})(\eta_{21} + \eta_{03})\right\}$$

$$I_7 = (3\eta_{21} - \eta_{03})(\eta_{30} + \eta_{12})\left\{(\eta_{30} + \eta_{12})^2 - 3(\eta_{21} + \eta_{03})^2\right\} +$$

$$(\eta_{30} - 3\eta_{12})(\eta_{21} + \eta_{03})\left\{3(\eta_{30} + \eta_{12})^2 - (\eta_{21} + \eta_{03})^2\right\}$$

$$\cdots\cdots (4)$$

For example, as is clear from Expressions (4), the first-order moment invariant $I_1$ is one in which variance toward the x-axis direction and variance toward the y-axis direction are added together. Incidentally, the moment invariants are provided by Hu (M-K Hu, Visual pattern recognition by moment invariants, IRE Trans. on Information Theory, IT-8, pp. 179-187, 1962).

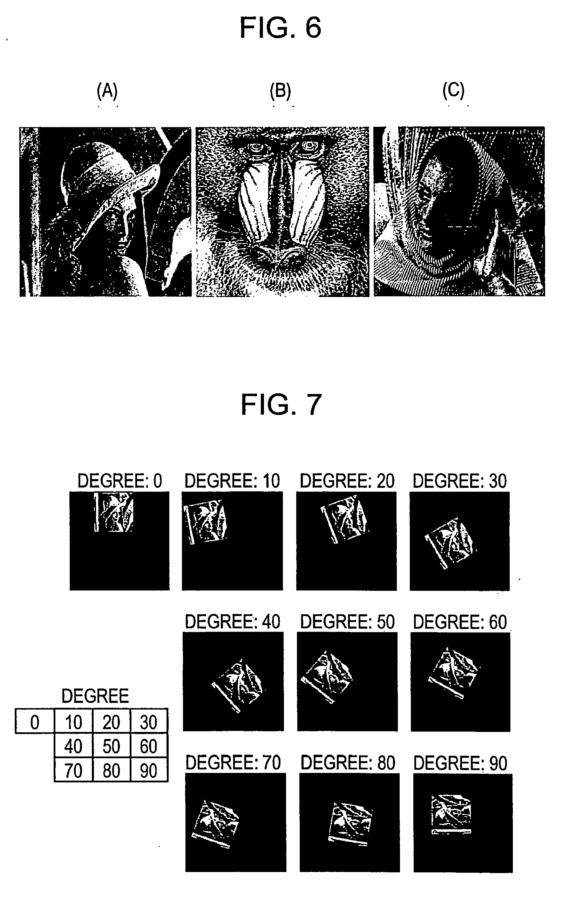

Here, when the sample images illustrated in Fig. 6 (Fig. 6(A): lenna, Fig. 6(B): mandrill, Fig. 6(C): barbara) were rotated every 10[°] with respect to the reference (angle 0[°]) and translated freely on a background where a pixel value is zero, as illustrated in Fig. 7, a moment invariant at each movement was calculated.

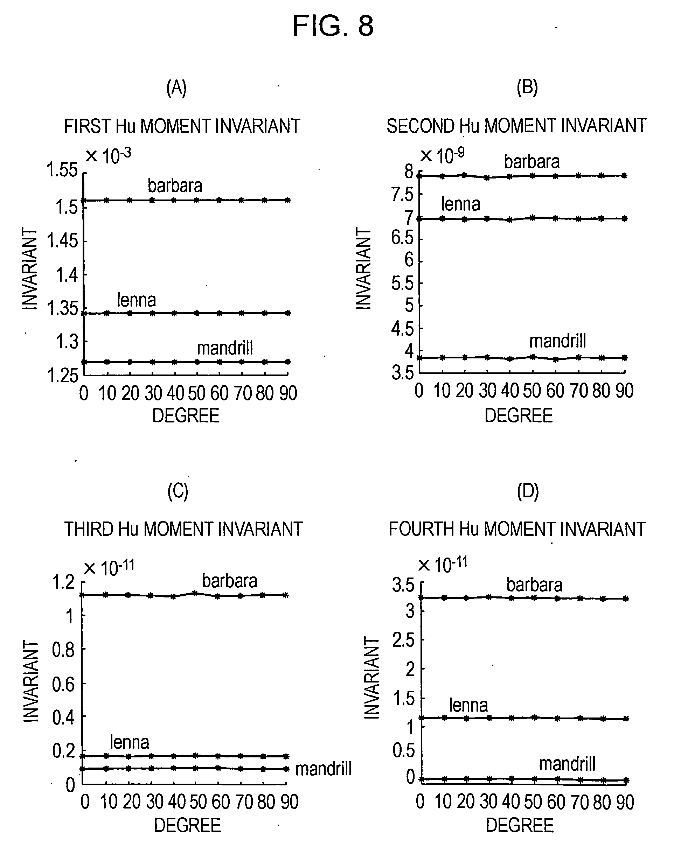

The results were illustrated in Fig. 8. This Fig. 8 plots relationships between moment invariants and degrees with the moment invariants being depicted in the vertical axis and the rotational degree being depicted in the horizontal axis. As is also clear from this Fig. 8, even under rotation movement and translation movement, the values of moment invariants are substantially uniform, that is, invariant.

Note that, as is also clear from Fig. 7, if not all the sample image is present within an image pickup range, the state of a pattern (relationship between a luminance value of a pixel and its position) of the sample image varies, so a moment invariant varies. Accordingly, when a target for extracting a moment invariant is the whole of a venous image, unless an image pickup range larger than an image pickup target is ensured, no movement of the image pickup target is permitted. That is, as a permissible range for movement of an image pickup target is extended, the size of the image pickup unit 12 is increased.

Consequently, for the feature extracting unit 43, a target for extracting a moment invariant is not the whole of a venous image, but is limited to part of the venous image. Due to this, the feature extracting unit 43 can permit movement of an image pickup target (on a finger placement surface, translation movement of a finger toward its surface direction or rotation movement of a finger about the longitudinal axis of the finger), irrespective of whether the image pickup range is large or small, as long as a range for extracting a moment invariant is within a venous image. As a result, the feature of an image pickup target (vein) can be properly caught, as compared with the case where a target for extracting a moment invariant is the whole of a venous image.

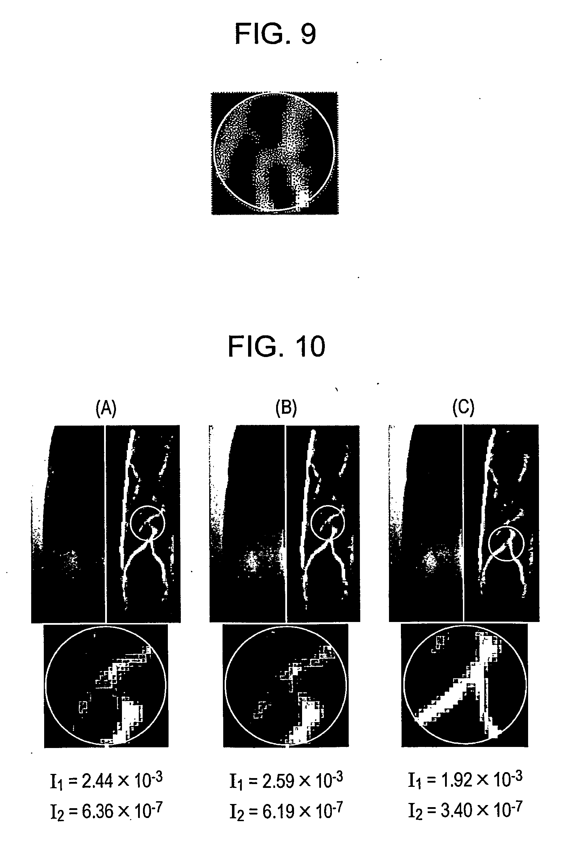

In the present embodiment, as a target for extracting a moment invariant, as illustrated in Fig. 9, a range whose center lies in a diverging point and includes its vicinity is used. Specifically, when the feature extracting unit 43 acquires image data from the sharpening unit 41, the feature extracting unit 43 also acquires position data on a diverging point of a vein in the image data from the reference-point detecting unit 42, and sets a circular extraction range whose center lies in a diverging point indicated by the position data and calculates a moment invariant.

Here, in Fig. 10, at an image pickup at the first time (Fig. 10(A)) and at an image pickup at the second time (Fig. 10(B) and Fig. 10(C)) when the image pickup position at the first time is moved in the surface direction of the finger placement surface (in the figure, moved rightward), venous images are shown in the left, venous images before and after sharpening processing are shown in the right, and enlarged images in a range for extracting a moment invariant are shown in the bottom.

As is also clear from this Fig. 10, in the extraction range whose center lies in the same diverging point (Fig. 10 (A) and Fig. 10(B)), even if the position of the finger on the finger placement surface is different between the vein registration mode and the authentication mode, both the first-order ($I_1$) and second-order ($I_2$) moment invariants are not different. In contrast, when the extraction range changes (Fig. 10(C)), both the first-order ($I_1$) and second-order ($I_2$) moment invariants significantly differ.

In this way, the feature extracting unit 43 can accurately calculate, in a vein, a moment invariant of a diverging section that greatly varies from person to person and is an important element for identification, irrespective of movement within a venous image.

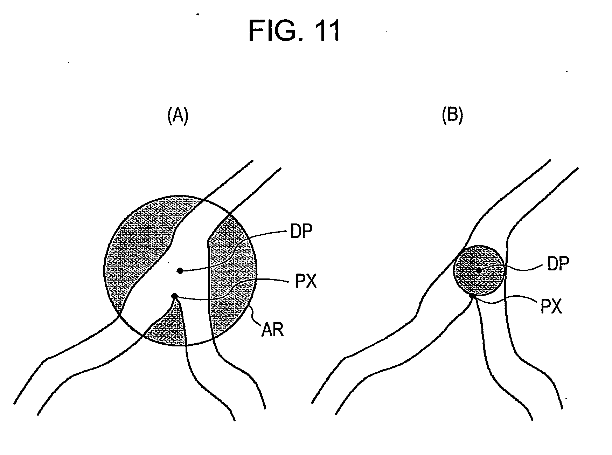

Meanwhile, when the feature extracting unit 43 sets an extraction range whose center lies in a diverging point, the extraction range contains the edge of the diverging section. This results from that, as illustrated in Fig. 11, the diverging point DP detected by the reference-point detecting unit 42 is a point of intersection occurring when the vein defines one pixel width, whereas the diverging section being the moment extraction target has any width.

That is, when an extraction range AR containing a diverging point PX on the outline is set (Fig. 11(A)), each diverging point on the venous image has traits in the position from the center and a luminance state at that position, whereas when the extraction range AR containing the diverging point PX is not set (Fig. 11(B)), the trait is weak and thus there is no significance as a target for identification.

It is to be noted that, when this extraction range is uniformly set, it is preferable that the radius of the extraction range be equal to or larger than a range being a unit of sharpening in the reference-point detecting unit 42 at the previous stage (kernel size). This is because, in that case, the possibility of containing the diverging point PX on the outline is high.

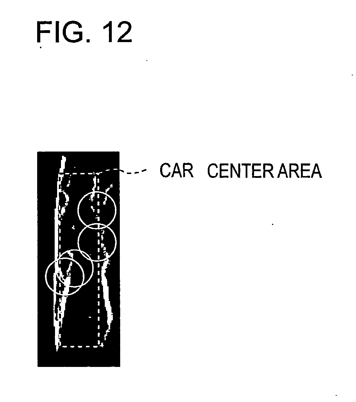

In addition, in the case of the present embodiment, as illustrated in Fig. 12, the feature extracting unit 43 uses only a diverging point contained in a central region extending from the center of a venous image to a certain distance as the target for extracting a moment invariant. Accordingly, the feature extracting unit 43 can further permit movement of an image pickup target, as compared with the case where moment invariant in the extraction range AR having each of all the diverging points contained in the venous image as the center is calculated.

(3) Details of Processing in Registration Unit

Next, the registration unit 23 is specifically described. This registration unit 23 acquires a moment invariant from the feature extracting unit 43 and determines whether the number of moment invariants calculated in the feature extracting unit 43 is equal to or larger than a prescribed number.

Here, when the number of moment invariants is smaller than the prescribed value, the registration unit 23 determines that it is insufficient as the feature of a registrant and notifies that an image should be picked up again through the notification unit 15.

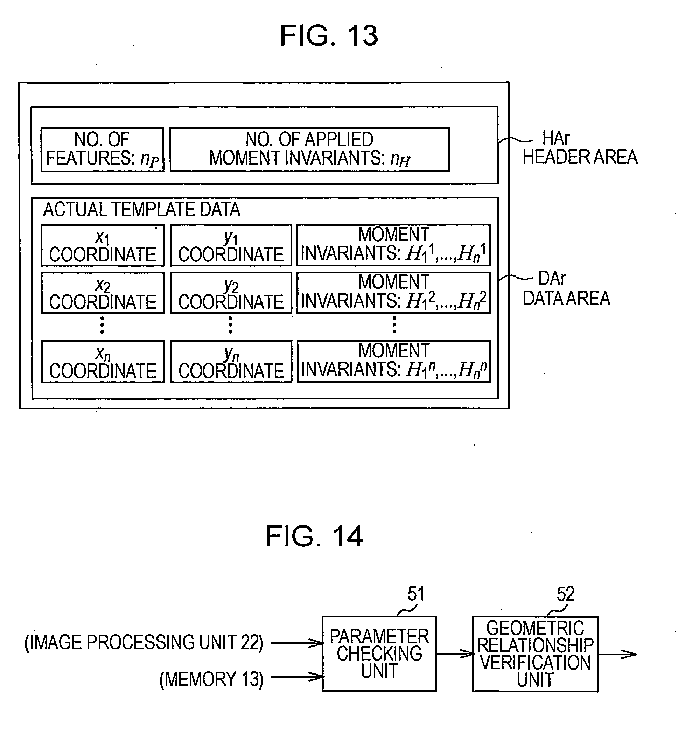

On the other hand, when the number of moment invariants is equal to or larger than the prescribed value, the registration unit 23 acquires position data on a diverging point of a vein in venous image data from the reference-point detecting unit 42 and generates registration data containing the position of the diverging point and a moment invariant calculated with reference to that position. The data structure of this registration data is illustrated in Fig. 13. This identification data has a structure containing a header area HAr and a data area DAr.

In the header area HAr, the number of diverging points and the number of applied orders among seven orders in Hu moment invariants are stored. These items specify the details of the processing in the image processing unit 22, so the content stored in the data area DAr has meaning as an identifier for confirming that the content stored in the data area DAr is a result of the processing in the image processing unit 22.

On the other hand, in the data area Dar, the position (xy coordinates) of a diverging point serving as a reference for extracting a moment invariant and a moment invariant H of an h-th order (h is 2, 3, ..., or 7) are stored in association with each other.

In such a way, the registration unit 23 registers, in the memory 13, registration data containing a result of the processing in the image processing unit 22 and the content that confirms that the result of the processing has been performed through the image processing unit 22.

(4) Details of Processing in Authentication Unit

Next, the authentication unit 32 is specifically described. The authentication unit 32 has a configuration that includes a parameter checking unit 51 and a geometric relationship verification unit 52.

(4-1) Parameter Checking Processing

The parameter checking unit 51 checks a moment invariant stored in the data area DAr (Fig. 13) of the registration data read from the memory 13 by the reading unit 31 (hereinafter referred to as a registration invariant) against a moment invariant extracted by the image processing unit 22 (Fig. 3) (hereinafter referred to as an authentication invariant).

Specifically, the parameter checking unit 51 calculates the deviation $d_I$ between each registration invariant and its corresponding authentication invariant for each the same order using the following expression:

$$ d_I = \frac{\left| I^T - I^S \right|}{I^T} \qquad \cdots\cdots (5) $$

where $I^T$ (T = 1, 2, ..., m (m is an integer)) is an i-th order (i = 1, 2, ..., 7) registration invariant in the data area DAr (Fig. 13) and $I^S$ (S = 1, 2, ..., n (n is an integer)) is an authentication invariant extracted by the image processing unit 22 (Fig. 3). Incidentally, the parameter checking unit 51 identifies a correlation between a registration invariant and an authentication invariant on the basis of a diverging point being a reference for extracting a moment invariant, for example.

And, when obtaining the deviations $d_I$ as a result of checking, the parameter checking unit 51 determines whether all of these deviations $d_I$ is smaller than a predetermined threshold. Here, when at least one of the deviations $d_I$ is equal to or larger than the threshold, this means that a venous image at registration and that at authentication are different, that is, the provider of a venous image at authentication is not a registrant. In this case, the parameter checking unit 51 notifies that the person cannot be authenticated as a registrant through the notification unit 15.

On the other hand, when all of the deviations $d_I$ is smaller than the predetermined threshold, this means that the possibility that the person who input a venous image is a registrant is high. In this case, the parameter checking unit 51 notifies the geometric relationship verification unit 52 that it should start processing.

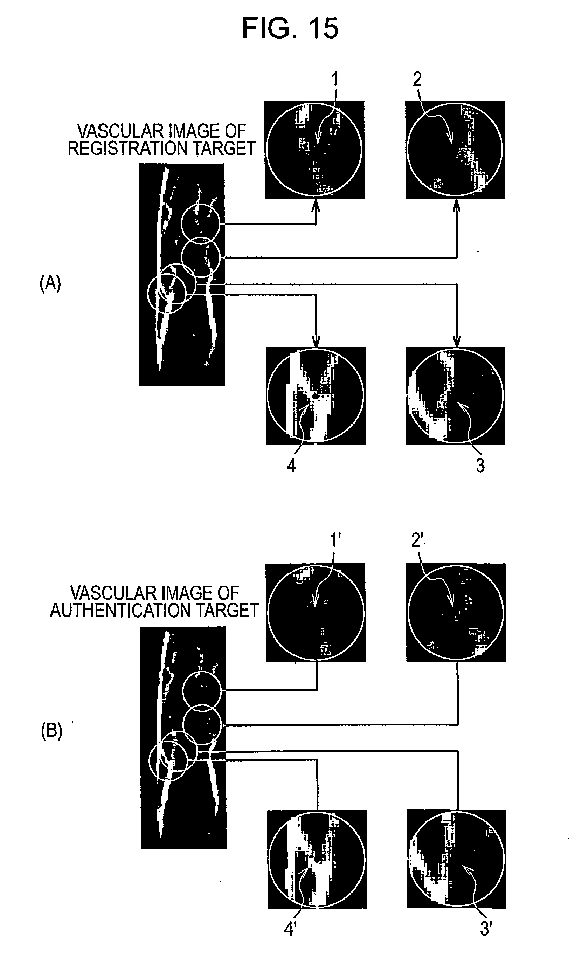

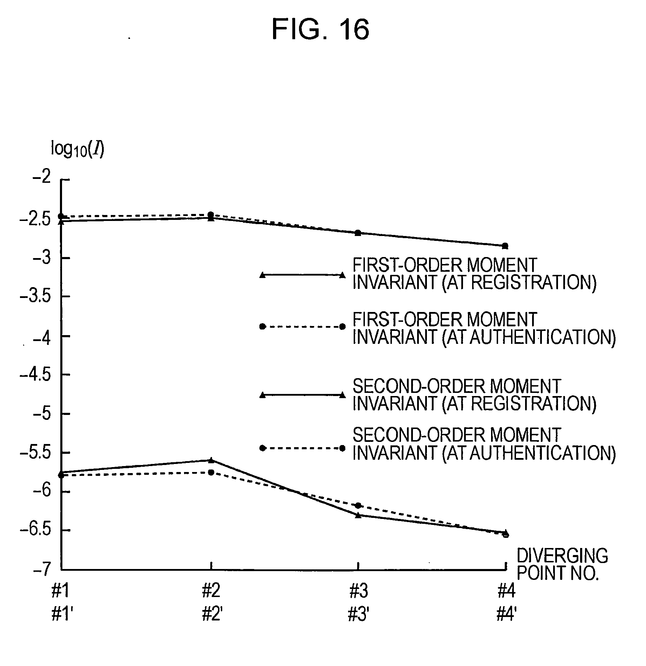

Here, a result of processing in the above-described parameter checking unit 51 using venous images illustrated in Fig. 15 as an example is illustrated in Fig. 16. In this Fig. 15, Fig. 15(A) illustrates a venous image acquired in the vein registration mode and an enlarged image at a section for use in calculating a registration invariant relating to that venous image, and Fig. 15(B) illustrates a venous image acquired from the same section for that registrant in the authentication mode and an enlarged image at a section for use in calculating an authentication invariant relating to that venous image.

And, the same numbers are assigned to corresponding diverging points, and among the diverging points, a diverging point being an authentication target has an apostrophe (') added to its number. Incidentally, in this Fig. 15, a venous section in a venous image acquired in the vein registration mode (Fig. 15(A)) and a venous section in a venous image acquired in the authentication mode (Fig. 15(B)) do not exist in reality, but they are illustrated for the sake of visual convenience.

Fig. 16 reveals that, because a registration invariant and an authentication invariant are acquired from the same section for the same person, these invariants substantially the same.

(4-2) Geometric Relationship Verification Processing

When being notified that it should start processing by the parameter checking unit 51, the geometric relationship verification unit 52 verifies whether the positional relationship between a diverging point being a reference point for extracting a registration invariant and a diverging point being a reference point for extracting an authentication invariant is in correlation.

Specifically, the geometric relationship verification unit 52 searches for a combination of a registration invariant and an authentication invariant at which the sum of squares of deviations is a minimum and determines whether the number of the combinations is equal to or larger than a prescribed number.

Here, when the number of the combinations is smaller than the prescribed number, this means that, because the number of correspondences between diverging points being reference points for extracting a registration invariant and diverging points being reference points for extracting an authentication invariant is small, there is no need to verify the positional relationship between the diverging points, and reliability is insufficient to authenticate the person as a registrant. In this case, the geometric relationship verification unit 52 notifies that the person cannot be authenticated as a registrant through the notification unit 15.

On the other hand, when the number of the combinations is equal to or larger than the prescribed number, the geometric relationship verification unit 52 acquires the position of a diverging point being a reference point for extracting a registration invariant and that for an authentication invariant in a combination that satisfies the condition that the minimum value of the sum of squares of deviations is smaller than a predetermined threshold. That is, the geometric relationship verification unit 52 acquires the position of the diverging point corresponding to the registration invariant from the data area DAr (Fig. 13) and acquires the position of the diverging point corresponding to the authentication invariant from the image processing unit 22.

Then, the geometric relationship verification unit 52 calculates the distances between the diverging points of one component in a combination and the distances between the diverging points of the other component in the combination and calculates the differences between these distances for each combination. In addition, the geometric relationship verification unit 52 divides the sum total of the differences between the distances calculated for each combination by the number of the combinations and sets the result of the division as an evaluated value for use in determining whether there is a geometric correlation.

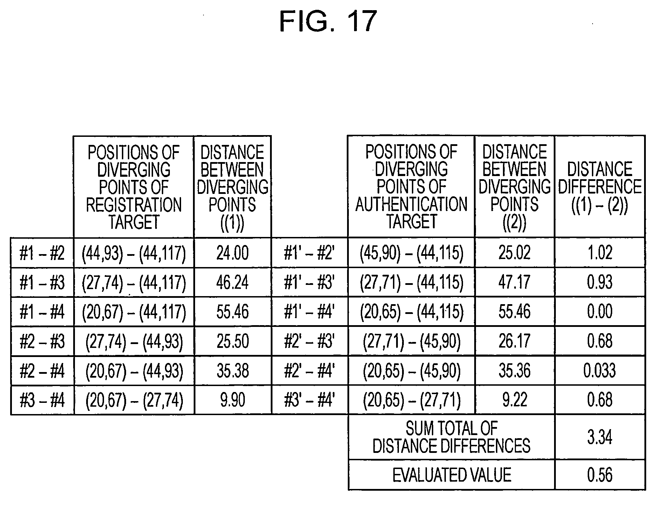

Here, a result of processing in the above-described geometric relationship verification unit 52 using the venous image illustrated in Fig. 15 as an example is illustrated in Fig. 17. In Fig. 17, the leftmost column indicates the combinations of numbers assigned to diverging points (i.e., combinations of diverging points) in a venous image acquired in the vein registration mode (Fig. 15(A)), and the fourth column counted from the leftmost column indicates the combinations of numbers assigned to diverging points in a venous image acquired in the authentication mode (Fig. 15(B)). On the other hand, the value in the column for an evaluated value indicates the result obtained by dividing the sum total of the differences between the distances by the number of the combinations (six).

And, when calculating an evaluated value, the geometric relationship verification unit 52 determines whether the evaluated value is smaller than a predetermined threshold. Here, when the evaluated value is equal to or larger than the predetermined threshold, this means that, although a registration invariant and an authentication invariant are not different, the diverging points being reference points for extracting these moment invariants are not in relative positional relation, so the shapes of veins in venous images being targets for extracting the registration invariant and the authentication invariant are different and the image acquired in the authentication mode is one other than an authorized venous image. In this case, the geometric relationship verification unit 52 notifies that the person cannot be authenticated as a registrant through the notification unit 15.

In contrast to this, when the evaluated value is smaller than the predetermined threshold, this means that a person who input the venous image in the authentication mode is a registrant. In this case, the geometric relationship verification unit 52 considers that the person can be authenticated as a registrant, generates data for starting execution of predetermined processing relating to successful authentication, and sends it to a device connected to the interface 14 (Fig. 1).

(5) Procedure of Authentication Processing

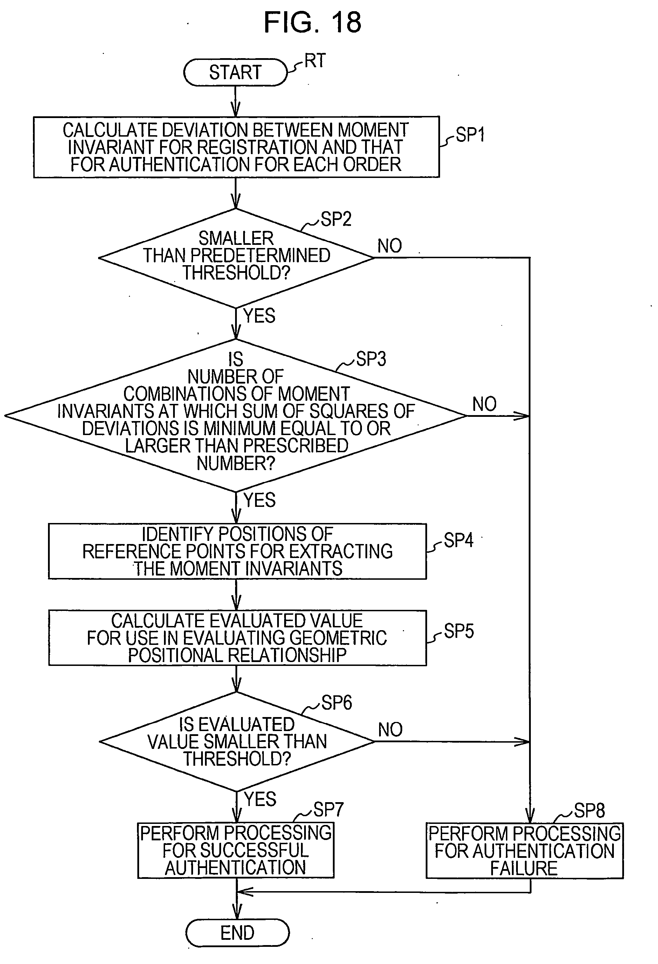

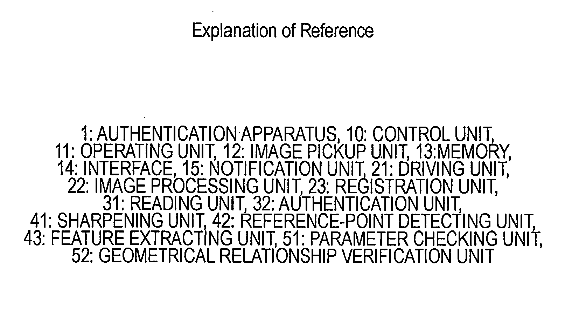

Next, a procedure of authentication processing in the authentication unit 32 is described using the flowchart of Fig. 18. That is, when receiving the command COM2 to execute the authentication mode from the operating unit 11, the authentication unit 32 starts this authentication processing procedure RT. In step SP1, the authentication unit 32 calculates a deviation between a registration invariant stored in the data area DAr (Fig. 13) and its corresponding authentication invariant for each order, and flow proceeds to step SP2.

In this step SP2, the authentication unit 32 determines whether all of the deviations calculated in step SP1 is smaller than a predetermined threshold. When all of the deviations is smaller than the predetermined threshold, flow proceeds to step SP3.

In this step SP3, the authentication unit 32 searches for a combination at which the sum of squares of deviations is a minimum and determines whether the number of the combinations is equal to or larger than a prescribed number. When the number of the combinations is equal to or larger than the prescribed number, flow proceeds to step SP4.

In this step SP4, the authentication unit 32 identifies the positions of diverging points being reference points for extracting a registration invariant and an authentication invariant in a combination that satisfies the condition that the minimum value of the sum of squares of deviations is smaller than a predetermined threshold. In the subsequent step SP5, the authentication unit 32 calculates an evaluated value based on a result of the identification.

That is, the authentication unit 32 calculates the distances between the diverging points for the registration invariant and the distances between the diverging points for the authentication invariant identified in step SP4, and after that, calculates the differences between the distances for each combination searched for in step SP3. Then, the authentication unit 32 sets, as an evaluated value, a value obtained by dividing the sum total of the differences between the distances calculated for each combination by the number of the combinations searched for in step SP3.

When calculating the evaluated value in step SP5, flow proceeds to step SP6, where the authentication unit 32 determines whether the evaluated value is smaller than a predetermined threshold.

Here, when the evaluated value is smaller than the predetermined threshold, the authentication unit 32 determines that the authentication is successful. In this case, flow proceeds to step SP7, where the authentication unit 32 performs predetermined processing associated with the successful authentication. After that, this authentication processing procedure RT is completed.

On the other hand, when the evaluated value is equal to or larger than the predetermined threshold, when in step SP2 at least one of the deviations between the registration invariants and the authentication invariants is equal to or larger than the predetermined threshold, or when in step SP3 the number of combinations of a registration invariant and an authentication invariant at which the sum of squares of deviations is a minimum is smaller than the prescribed number, the authentication unit 32 determines that the authentication fails. In this case, flow proceeds to step SP8, where the authentication unit 32 performs predetermined processing associated with the authentication failure. After that, this authentication processing procedure RT is completed.

In such a way, the authentication unit 32 can determine whether a person is a registrant by comparing values of features (moment invariants, positional information) without image matching processing.

(6) Operation and Effect

In the above configuration, this authentication apparatus 1 extracts a feature having a parameter resistant to affine transformation from only a diverging section of a vein shown in a venous image (for example, Fig. 10). This enables the authentication apparatus 1 to extract an equivalent feature without an increase in the image pickup range in the image pickup unit 12 even if the vein moves within the venous image.

Accordingly, this authentication apparatus 1 can more prevent a decrease in authentication accuracy while maintaining the permissible amount of movement of a vein within a venous image, as compared with the case where a feature is extracted from the whole of a venous image. This is particularly useful in the case where it is incorporated in a device whose miniaturization is highly desired, such a cellular phone.

In the case of the present embodiment, the use of a moment invariant as a feature enables extraction of an equivalent feature even if a finger is translated toward in its surface direction on the finger placement surface and/or the finger is rotated about the longitudinal axis of the finger.

And, in the case of the present embodiment, an extraction range is set so as to have, as its center, among diverging sections of a vein shown in a venous image, a diverging section contained in a central area CAR (Fig. 12) that is distant from the central position of the venous image by a certain distance, and a feature is extracted from each of the set extraction ranges.

Accordingly, this authentication apparatus 1 can further maintain the permissible amount of movement of a vein in a venous image, as compared with the case where a target for extracting a feature is not limited to the central area CAR. And, it is particularly useful in the case where the structure of the image pickup unit 12 is not the one in which a light source and an image pickup element are arranged so as to sandwich a finger placed on a finger placement surface (for example, Fig. 15 in Japanese Unexamined Patent Application Publication No. 2005-353014) but the one in which a light source and an image pickup element are arranged in the same direction with respect to the placement surface of the finger placed on the finger placement surface where the finger is placed (for example, Fig. 2 in the same publication).

This is because, in the case of the structure in which the light source and the image pickup element are arranged with respect to the placement surface of the finger, visible light may be mixed in near-infrared light emitted from the light source to the vein, from the end of that finger placement surface as noise, and the end of the venous image that is difficult to be shown because of this can be removed from the target for extracting a feature.

With the above configuration, the extraction of a feature having a parameter resistant to affine transformation from only a diverging section of a vein shown in a venous image enables the permissible amount of movement of the vein in the venous image without an increase in the image pickup range to be maintained. In this manner, the authentication apparatus 1 capable of achieving an improved authentication accuracy can be accomplished.

(7) Other Embodiments

In the above-described embodiment, the case where a LoG filter is used in sharpening processing in the sharpening unit 41 is described. However, the present invention is not limited to this case. For example, various filters, such as a morphology filter, Laplacian filter, a Gaussian filter, or a Sobel filter, can also be used. And, the number of filters used may also be more than one.

And, in the above-described embodiment, the case where a diverging point is used as a detection target in the reference-point detecting unit 42 is described. However, the present invention is not limited to this case. Either an end point or an inflection point, or a combination of both may also be used. Alternatively, for example, among points included in a vein, a point, such as a point that intersects a circle whose center lies in the center of the venous image, other than all or part of an end point, a diverging point, and an inflection point may also be used.

In addition, although the case where the reference-point detecting unit 42 detects a detection target fixed at a diverging point is described, the present invention is not limited to this case. The detection target may also be switched at a predetermined timing.

For example, every time the vascular registration mode is executed, the detection target is switched among a diverging point, an inflection point, and a point that intersects a circle whose center lies in the central position of the image in this order. Alternatively, for example, every time the vascular registration mode is executed, the radius of a circle whose center is the central position is switched and a point that intersects the circle is detected. In this case, describing an identifier for identifying how it is detected (that is, a detection technique) in the header area HAr (Fig. 13) and, in the authentication mode, detecting a detection target by the image processing unit 22 (Fig. 3) using the detection technique indicated by the identifier enables extraction of moment invariants using corresponding points as references at both registration and authentication.

With this, even in the event that registered information on a vein is stolen in another system, because the information does not reveal how a reference point for a moment invariant is detected, this authentication apparatus 1 can prevent masquerading as a registrant using the stolen information, so authentication accuracy can be further improved.

In addition, in the above-described embodiment, the case where Hu moment invariants are used as an extraction target in the feature extracting unit 43 is described. However, the present invention is not limited to this case. For example, Zernike moments or entropy may also be used. And, it is possible to use one in which an invariant for only translation movement, like a local luminance histogram in a venous image, not an invariant for both translation movement of a finger toward its surface direction on the finger placement surface and rotation movement of the finger about the longitudinal axis of the finger, like a moment invariant. In short, various features can be used as long as they are resistant to affine transformation.

In addition, in the above-described embodiment, the case where the authentication apparatus 1 having the image pickup function, checking function, and registration function is used is described. However, the present invention is not limited to this case. A mode in which each function or part of the functions is assigned to a single device depending on the application may also be used.

Industrial Applicability

The present invention is applicable in the field of biometrics authentication.

**Claims**

1. A registration apparatus **characterized by** comprising:

   an image acquisition unit configured to acquire a venous image for a vein of a living body;
   an extraction unit configured to extract a parameter resistant to affine transformation from part of the venous image; and
   a registration unit configured to register the parameter extracted by the extraction unit in storage means.

2. The registration apparatus according to Claim 1, **characterized in that** the extraction unit is configured to set an extraction range to each of diverging sections of a vein shown in the venous image such that the extraction range contains an edge of the diverging section and configured to extract the parameter from each of the set extraction ranges.

3. The registration apparatus according to Claim 1, **characterized in that** the extraction unit is configured to set an extraction range whose center lies in, among diverging sections of a vein shown in the venous image, a diverging section contained in a central region whose reference lies in a central position of the venous image and configured to extract the parameter from each of the set extraction ranges.

4. The registration apparatus according to Claim 1, **characterized in that** it further comprises a detection unit configured to detect a plurality of venous section points from a vein shown in the venous image, and

the extraction unit is configured to extract the parameter from a predetermined extraction range whose center lies in each of the venous section points.

5. The registration apparatus according to Claim 4, **characterized in that** the detection unit is configured to detect, as the plurality of venous section points, all or part of an end point, a diverging point, and an inflection point of the vein.

6. The registration apparatus according to Claim 4, **characterized in that** the detection unit is configured to switch a detection technique of detecting the plurality of venous section points at a predetermined timing, and
the registration unit is configured to register the parameter and an identifier for identifying the detection technique in the storage means.

7. The registration apparatus according to Claim 1, **characterized in that** the parameter is a moment invariant.

8. An authentication apparatus **characterized by** comprising:

an extraction unit configured to extract a parameter resistant to affine transformation from part of a venous image for a vein of a living body, the venous image being input as an authentication target;
a reading unit configured to read registration information registered in storage means; and
a determination unit configured to determine whether a person who input the parameter is a registrant in accordance with a degree of checking between the parameter and the registration information.

9. The authentication apparatus according to Claim 8, **characterized in that** the determination unit is configured to determine that the person who input the parameter is a registrant when a deviation between the parameter and a corresponding parameter in the registration information is smaller than a predetermined threshold.

10. The authentication apparatus according to Claim 8, **characterized in that** the extraction unit is configured to set an extraction range to each of diverging sections of a vein shown in the venous image and configured to extract the parameter from each of the set extraction ranges, and
the determination unit is configured to determine that the person who input the parameter is a registrant when the deviations between the parameters and the corresponding parameters in the registration information are smaller than the predetermined threshold and when a diverging section to which the extraction range of each of the parameters is set and a diverging section to which the extraction range of each of the corresponding parameters in the registration information is set are geometrically correlated.

11. A data structure for use in processing of determination of a registrant or not performed by a computer, the data structure being **characterized in that** it includes a parameter extracted from part of a venous image for a vein of the registrant, the parameter being resistant to affine transformation, and
in the processing, the parameter is compared with a corresponding section in a venous image input as an authentication target.

12. The data structure according to Claim 11, **characterized in that** it includes positional information on the part, in addition to the parameter, and
in the processing, it is determined whether the positional information is geometrically correlated with positional information on the corresponding section.

13. A storage medium that stores data for use in processing of determination of a registrant or not performed by a computer, the storage medium being **characterized in that** the data includes a parameter extracted from part of a venous image for a vein of the registrant, the parameter being resistant to affine transformation, and
in the processing, the parameter is compared with a corresponding section in a venous image input as an authentication target.

14. The storage medium according to Claim 13, **characterized in that** it includes positional information on the part, in addition to the parameter, and
in the processing, it is determined whether the positional information is geometrically correlated with positional information on the corresponding section.

# FIG. 1

COM1,COM2

1

| 12 IMAGE PICKUP UNIT | 10 CONTROL UNIT | 11 OPERATING UNIT |

13 MEMORY

14 INTERFACE

DISPLAY UNIT 15a

AUDIO OUTPUT UNIT 15b

16 BUS

15 NOTIFICATION UNIT

# FIG. 2

12 IMAGE PICKUP UNIT

10 CONTROL UNIT

21 DRIVING UNIT

22 IMAGE PROCESSING UNIT

23 REGISTRATION UNIT

# FIG. 3

# FIG. 4

22

```
      41                    42                    43
 ┌──────────┐      ┌──────────────┐      ┌────────────┐
─→│SHARPENING│─→   │REFERENCE-POINT│─→   │  FEATURE   │─→
 │   UNIT   │      │  DETECTING   │      │ EXTRACTING │
 └──────────┘      │     UNIT     │      │    UNIT    │
                   └──────────────┘      └────────────┘
```

# FIG. 5

(A) BEFORE
SHARPENING

(B) AFTER
SHARPENING

# FIG. 6

(A)                    (B)                    (C)

# FIG. 7

DEGREE: 0    DEGREE: 10    DEGREE: 20    DEGREE: 30

DEGREE: 40    DEGREE: 50    DEGREE: 60

| DEGREE | | | |
|---|---|---|---|
| 0 | 10 | 20 | 30 |
| | 40 | 50 | 60 |
| | 70 | 80 | 90 |

DEGREE: 70    DEGREE: 80    DEGREE: 90

# FIG. 8

(A)

FIRST Hu MOMENT INVARIANT

(B)

SECOND Hu MOMENT INVARIANT

(C)

THIRD Hu MOMENT INVARIANT

(D)

FOURTH Hu MOMENT INVARIANT

## FIG. 9

## FIG. 10

(A)                          (B)                          (C)

$I_1 = 2.44 \times 10^{-3}$

$I_2 = 6.36 \times 10^{-7}$

$I_1 = 2.59 \times 10^{-3}$

$I_2 = 6.19 \times 10^{-7}$

$I_1 = 1.92 \times 10^{-3}$

$I_2 = 3.40 \times 10^{-7}$

# FIG. 11

(A)

(B)

FIG. 12

CAR CENTER AREA

# FIG. 13

| NO. OF FEATURES: $n_P$ | NO. OF APPLIED MOMENT INVARIANTS: $n_H$ | | ~ HAr HEADER AREA |
|---|---|---|---|

ACTUAL TEMPLATE DATA

| $x_1$ COORDINATE | $y_1$ COORDINATE | MOMENT INVARIANTS: $H_1^1,...,H_n^1$ |
|---|---|---|
| $x_2$ COORDINATE | $y_2$ COORDINATE | MOMENT INVARIANTS: $H_1^2,...,H_n^2$ |
| $\vdots$ | $\vdots$ | $\vdots$ |
| $x_n$ COORDINATE | $y_n$ COORDINATE | MOMENT INVARIANTS: $H_1^n,...,H_n^n$ |

~ DAr DATA AREA

# FIG. 14

(IMAGE PROCESSING UNIT 22) ⟶

(MEMORY 13) ⟶

51
PARAMETER CHECKING UNIT ⟶

52
GEOMETRIC RELATIONSHIP VERIFICATION UNIT ⟶

# FIG. 15

# FIG. 16

# FIG. 17

| | POSITIONS OF DIVERGING POINTS OF REGISTRATION TARGET | DISTANCE BETWEEN DIVERGING POINTS ((1)) | | POSITIONS OF DIVERGING POINTS OF AUTHENTICATION TARGET | DISTANCE BETWEEN DIVERGING POINTS ((2)) | DISTANCE DIFFERENCE ((1) – (2)) |
|---|---|---|---|---|---|---|
| #1 – #2 | (44,93) – (44,117) | 24.00 | #1' – #2' | (45,90) – (44,115) | 25.02 | 1.02 |
| #1 – #3 | (27,74) – (44,117) | 46.24 | #1' – #3' | (27,71) – (44,115) | 47.17 | 0.93 |
| #1 – #4 | (20,67) – (44,117) | 55.46 | #1' – #4' | (20,65) – (44,115) | 55.46 | 0.00 |
| #2 – #3 | (27,74) – (44,93) | 25.50 | #2' – #3' | (27,71) – (45,90) | 26.17 | 0.68 |
| #2 – #4 | (20,67) – (44,93) | 35.38 | #2' – #4' | (20,65) – (45,90) | 35.36 | 0.033 |
| #3 – #4 | (20,67) – (27,74) | 9.90 | #3' – #4' | (20,65) – (27,71) | 9.22 | 0.68 |
| | | | | SUM TOTAL OF DISTANCE DIFFERENCES | | 3.34 |
| | | | | EVALUATED VALUE | | 0.56 |

# FIG. 18

START ⟩⟋RT

CALCULATE DEVIATION BETWEEN MOMENT
INVARIANT FOR REGISTRATION AND THAT
FOR AUTHENTICATION FOR EACH ORDER ⟋SP1

SP2
SMALLER
THAN PREDETERMINED
THRESHOLD? — NO

YES

SP3
IS
NUMBER OF
COMBINATIONS OF MOMENT
INVARIANTS AT WHICH SUM OF SQUARES OF
DEVIATIONS IS MINIMUM EQUAL TO OR
LARGER THAN PRESCRIBED
NUMBER? — NO

YES

IDENTIFY POSITIONS OF
REFERENCE POINTS FOR EXTRACTING
THE MOMENT INVARIANTS ⟋SP4

CALCULATE EVALUATED VALUE
FOR USE IN EVALUATING GEOMETRIC
POSITIONAL RELATIONSHIP ⟋SP5

SP6
IS EVALUATED
VALUE SMALLER THAN
THRESHOLD? — NO

YES

SP7
PERFORM PROCESSING
FOR SUCCESSFUL
AUTHENTICATION

SP8
PERFORM PROCESSING
FOR AUTHENTICATION
FAILURE

END

Explanation of Reference

1: AUTHENTICATION APPARATUS, 10: CONTROL UNIT,
11: OPERATING UNIT, 12: IMAGE PICKUP UNIT, 13:MEMORY,
14: INTERFACE, 15: NOTIFICATION UNIT, 21: DRIVING UNIT,
22: IMAGE PROCESSING UNIT, 23: REGISTRATION UNIT,
31: READING UNIT, 32: AUTHENTICATION UNIT,
41: SHARPENING UNIT, 42: REFERENCE-POINT DETECTING UNIT,
43: FEATURE EXTRACTING UNIT, 51: PARAMETER CHECKING UNIT,
52: GEOMETRICAL RELATIONSHIP VERIFICATION UNIT

**INTERNATIONAL SEARCH REPORT**

| International application No. |
|---|
| PCT/JP2008/062875 |

A. CLASSIFICATION OF SUBJECT MATTER
*G06T7/00*(2006.01)i

According to International Patent Classification (IPC) or to both national classification and IPC

B. FIELDS SEARCHED

Minimum documentation searched (classification system followed by classification symbols)
G06T7/00

Documentation searched other than minimum documentation to the extent that such documents are included in the fields searched
Jitsuyo Shinan Koho          1922–1996   Jitsuyo Shinan Toroku Koho   1996–2008
Kokai Jitsuyo Shinan Koho   1971–2008   Toroku Jitsuyo Shinan Koho   1994–2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2006-53773 A  (Sony Corp.),<br>23 February, 2006 (23.02.06),<br>Par. Nos. [0028] to [0032]<br>& US 2006/0034501 A1     & CN 1737823 A | 1,7-9,11,13<br>2-6,10,12,14 |
| Y<br>A | JP 6-131444 A  (Masanori SUGISAKA),<br>13 May, 1994 (13.05.94),<br>Par. No. [0009]<br>(Family: none) | 1,7-9,11,13<br>2-6,10,12,14 |
| A | JP 2-48776 A  (Fujitsu Ltd.),<br>19 February, 1990 (19.02.90),<br>Claim 1<br>(Family: none) | 2-6,10,12,14 |

☐ Further documents are listed in the continuation of Box C.          ☐ See patent family annex.

| | |
|---|---|
| * Special categories of cited documents:<br>"A"  document defining the general state of the art which is not considered  to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| 05 August, 2008 (05.08.08) | 12 August, 2008 (12.08.08) |

| Name and mailing address of the ISA/<br>   Japanese Patent Office | Authorized officer |
|---|---|
| Facsimile No. | Telephone No. |

Form PCT/ISA/210 (second sheet) (April 2007)

# EP 2 169 621 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005312748 A

- JP 2005353014 A

### Non-patent literature cited in the description

- **M-K Hu.** Visual pattern recognition by moment invariants. *IRE Trans. on Information Theory,* 1962, vol. IT-8, 179-187